# FASCICULE DE BREVET EUROPEEN

(11) **EP 0 810 821 B1**
(45) Date de publication et mention de la délivrance du brevet: **20.01.1999**
(21) Numéro de dépôt: 96904889.1
(22) Date de dépôt: 21.02.1996
(51) Int. Cl.: A01N 1/02, A61K 35/52

(54) **PROCEDE DE DILUTION ET DE CONSERVATION DU SPERME DE LAPIN**
VERFAHREN ZUR VERDÜNNUNG UND KONSERVIERUNG VON KANINCHENSPERMIEN
METHOD FOR DILUTING AND PRESERVING RABBIT SEMEN

(30) Priorité: 23.02.1995 HU 9500562
(43) Date de publication de la demande: 10.12.1997
(73) Titulaire: AGRIBRANDS EUROPE FRANCE, 49160 Longue Jumelles (FR)
(72) Inventeur: SINKOVICS, György, H-6636 Martély (HU)
(74) Mandataire: Simonnot, Bernard
(86) Numéro de dépôt international: FR9600277
(87) Numéro de publication internationale: WO9625848

(56) Documents cités:
- DD-A- 58 359
- GB-A- 1 061 336
- CHEMICAL ABSTRACTS, vol. 74, no. 19, 1971 Columbus, Ohio, US; abstract no. 95166y, M.V. SILAEVA: "Medium for the dilution and storage of boar sperm." XP002008505 & VETERINARIYA (MOSCOW), vol. 47, no. 12, 1970, pages 81-83,
- CHEMICAL PATENTS INDEX, BASIC ABSTRACTS JOURNAL Week 8744 Derwent Publications Ltd., London, GB; AN 87-312123 XP002008506 & SU,A,1 291 140 (POULTRY TECH. RES. IN.)

## Description

La présente invention concerne un procédé de dilution et de conservation du sperme de lapin.

Dans ce domaine, on ne connaît à ce jour que deux méthodes de diffusion du sperme de lapin :
- le sperme frais,
- le sperme congelé.

Or ces deux méthodes présentent toutes deux des inconvénients majeurs :
- la première pose d'importants problèmes de logistique puisque le sperme frais ne conserve son pouvoir fécondant que pendant une durée maximale de 8 heures après avoir été prélevé,
- la seconde permet de conserver le sperme pour une durée indéterminée, mais les difficultés surgissent au moment de la décongélation, opération particulièrement délicate puisque le taux d'échec est très élevé (ce qui rend actuellement impossible l'utilisation pratique de cette méthode).

Par ailleurs, il faut noter que les méthodes de conservation du sperme d'autres espèces animales comme le bovin ou le porc ne sont en rien transposables au sperme de lapin. Aucune extrapolation interspécifique n'est possible. La preuve en est que la cryoconservation du sperme de bovin est parfaitement maîtrisée et constitue une réussite alors que ce procédé ne permet que des résultats extrêmement faibles en matière d'insémination artificielle du lapin.

Plus précisément, il est connu que la congélation et la conservation du sperme dilué de l'homme et de certains animaux domestiques, par exemple le taureau, à la température de l'azote liquide, -195°C, sont maîtrisés depuis des décennies.

Cependant, le sperme dilué de lapin domestique ne peut pas être stocké par congélation sans détérioration et, selon les données bibliographiques, les taux de fécondation sont plus faibles avec le sperme congelé qu'avec le sperme frais.

Dans la situation actuelle de l'élevage de lapin au niveau hongrois en particulier et européen en général, la technique de conservation du sperme de lapin par congélation n'a pas d'intérêt pratique réel, car la production de la viande de lapin et la production de la laine angora sont réalisées en premier lieu dans de petites exploitations qui ne sont pas aptes à la réception et à la conservation du sperme congelé.

Le problème est encore compliqué par le fait que, dans le cas du sperme congelé de lapin, la technique de la décongélation est un élément essentiel de l'utilisation du sperme et qu'actuellement on ne la réalise que dans les instituts de recherche, par des moyens contrôlés par ordinateur.

Les recherches qui ont conduit à l'invention ont montré de façon surprenante que l'on pouvait remédier aux inconvénients précités et apporter une solution aux problèmes évoqués grâce à une conception nouvelle du diluant, qui permet de conserver le sperme de lapin fraîchement prélevé pendant deux à trois jours, sans congélation, tout en maintenant son pouvoir fécondant.

Conformément à l'invention, le procédé de dilution et de conservation du sperme de lapin est du type dans lequel on prépare un diluant liquide citraté contenant, pour 100 ml d'eau distillée stérile, environ 2,00 à 2,10 g de citrate de trisodium, 1,3 à 1,6 g de glucose D et 5 à 15 ml de jaune d'oeuf battu séparément.

Selon une caractéristique essentielle de l'invention, on ajoute au diluant liquide citraté environ 1 à 3 ml de gélatine ordinaire mise à l'état liquide par chauffage préalable jusqu'à environ 45 à 55°C, on laisse la température du mélange liquide s'abaisser jusqu'à environ 34 à 40°C et l'on ajoute alors le sperme de lapin fraîchement prélevé, on laisse enfin la température s'abaisser jusqu'à atteindre une température de stockage ou de transport d'environ O à 15°C, pour laquelle la gélatine ajoutée s'est entièrement solidifiée en gelée, fixant ainsi les spermatozoïdes mobiles, de sorte qu'ils ne perdent pas d'énergie et conservent leur pouvoir fécondant pendant deux à trois jours, le mélange solidifié en gelée pouvant être transporté sans détérioration des spermatozoïdes, même en cas de secousses, de mouvements ou de cahotements liés aux conditions habituelles de transport.

Selon une autre caractéristique essentielle de l'invention, après avoir ajouté le sperme de lapin au mélange liquide dont la température est encore d'environ 34 à 40°C, on laisse la température dudit mélange liquide s'abaisser jusqu'à environ 28 à 37°C, on fait alors barboter pendant quelques secondes du dioxyde de carbone dans le mélange liquide, à raison d'environ 5 à 15 cm³ de dioxyde de carbone pour 100 ml de sperme dilué puis, après barbotage, on ferme immédiatement le récipient contenant le sperme de lapin dilué et on laisse le mélange se refroidir jusqu'à solidification de la gélatine en gelée, le dioxyde de carbone éliminant l'oxygène contenu dans le mélange à l'état liquide et favorisant ainsi la conservation du sperme en condition d'anaérobie pendant 72 heures, la diminution de l'oxygène disponible entraînant un ralentissement supplémentaire de l'activité métabolique des spermatozoïdes tout en assurant la stabilité chimique du milieu à une valeur proche du pH normal du sperme de lapin.

Selon d'autres caractéristiques de l'invention:
- on prépare un diluant liquide citraté contenant de préférence environ 2,03 g de citrate de trisodium, 1,5 g de glucose D et 10 ml de jaune d'oeuf pour 100 ml d'eau distillée stérile,
- on ajoute au diluant liquide citraté de préférence environ 2 ml de gélatine ordinaire liquéfiée de préférence à environ 50°C,
- on laisse la température du mélange liquide s'abaisser de préférence jusqu'à environ 37°C, qui est la température moyenne normale du sperme de lapin fraîchement prélevé,
- après avoir ajouté le sperme de lapin au mélange liquide dont la température est de préférence d'environ 37°C, on laisse la température du mélange liquide s'abaisser jusqu'à une température qui est de préférence d'environ 30 à 35°C, température à laquelle on réalise le barbotage du dioxyde de carbone,
- on fait barboter le dioxyde de carbone dans le mélange liquide, de préférence à raison d'environ 10 cm³ pour 100 ml de sperme dilué, par exemple au moyen d'une seringue,
- on stocke ou on transporte le mélange solidifié en gelée de préférence à une température comprise entre 5 et 10°C.

D'autres caractéristiques et avantages de l'invention ressortiront mieux de la description qui va suivre.

On s'est fixé comme objectif l'élaboration d'un diluant de sperme de lapin, dans lequel le sperme du lapin peut être stocké à la température du réfrigérateur pendant deux à trois jours sans perdre son pouvoir fécondant.

L'essence de l'invention est le procédé, par lequel au diluant citraté du sperme de lapin traditionnellement employé à large échelle (composition : 100 ml d'eau distillée stérile additionnée de 2,00 à 2,10 g, de préférence 2,03 g, de citrate de trisodium, de 1,3 g à 1,6 g, de préférence 1,5 g, de glucose D et de 5 à 15 ml, de préférence 10 ml, de jaune d'oeuf battu séparément), on ajoute une quantité de 1 à 3 ml, de préférence 2 ml, de gélatine ordinaire (substance produite par l'hydrolyse, lors de la cuisson dans l'eau, de la peau, des tissus conjonctifs, des protéines des os et du cartilage notamment, et pouvant être achetée dans le commerce), ce mélange étant amené à une température comprise entre 45°C et 55°C, de préférence 50°C.

Puis on laisse la température du mélange ainsi réalisé se refroidir jusqu'à une valeur comprise entre 34°C et 40°C, de préférence 37°C (le mélange restant fluide) et l'on y ajoute le sperme de lapin fraîchement prélevé. On obtient ainsi le sperme dilué de lapin.

Lorsque la température du sperme dilué est descendue jusqu'à une valeur comprise entre 28 et 37°C, de préférence entre 30 et 35°C, on fait barboter du dioxyde de carbone, par exemple à l'aide d'une seringue, pendant quelques secondes. La quantité de dioxyde de carbone est de 5 à 15 cm³, de préférence 10 cm³, pour 100 ml de sperme de lapin dilué. Après ce barbotage, on ferme immédiatement le récipient contenant le sperme de lapin dilué.

La conservation du sperme de lapin ainsi dilué peut être effectuée à la température normale d'un réfrigérateur (de 0°C à 15°C, de préférence de 5°C à 10°C). A la température du réfrigérateur, la gélatine est solidifiée en gelée, et elle fixe les spermatozoïdes mobiles, de sorte qu'ils ne perdent pas leur énergie et conservent leur pouvoir fécondant pendant deux à trois jours. La gélatine solidifie le sperme dilué, qui devient ainsi apte au transport (malgré les cahotements, mouvements, chocs) sans que les spermatozoïdes ne se détériorent.

Au cours de l'élaboration de l'invention, on a effectué des recherches bibliographiques rétrospectives sur ce thème dans la base de données CAB CD (Centre for Agriculture and Biosciences International), à partir de 1990. Aucune publication ne mentionne une telle utilisation de la gélatine, à propos d'un animal zootechnique. Et si, parallèlement aux essais en relation avec l'étude du sperme, la gélatine a été mentionnée, c'est uniquement parce qu'elle a été employée en tant que support pour la démonstration de réactions immunologiques.

### ESSAIS DE VERIFICATIONS DU DILUANT CONTENANT DE LA GELATINE POUR DU SPERME DE LAPIN

On a contrôlé le sperme de lapin dilué dans le diluant contenant de la gélatine par les méthodes habituelles d'examens microscopiques de sperme, puis on a vérifié son efficacité par des inséminations artificielles effectuées avec le sperme conservé selon l'invention.

### Matériels et méthodes :

On a effectué les essais en utilisant des lapins mâles de races et de lignées diverses. On a prélevé le sperme à l'aide d'un vagin artificiel, puis on a dilué le sperme, soit dans des diluants contenant de la gélatine, soit dans d'autres diluants utilisés de façon classique pour l'insémination artificielle du lapin, en général dans la proportion d'1 dose de sperme dans 5 à 20 doses, de préférence 10 doses, de diluant.

Après la dilution, on a stocké les échantillons dans le réfrigérateur, et l'on a évalué la qualité du sperme par observation au microscope après 8, 24, 48, puis 72 heures. Au cours de l'appréciation, on a calculé les proportions de spermatozoïdes mobiles et immobiles, ainsi que le sens de mouvement et les caractéristiques des spermatozoïdes vivants. Au cours des essais, on a également effectué des inséminations avec du sperme conservé dans le diluant contenant de la gélatine pendant deux à trois jours, et l'on a comparé les résultats avec ceux des inséminations effectuées à partir de sperme frais, dans des élevages de lapins de petits producteurs.

En outre, on a transporté et expédié sur de longues distances le sperme conservé dans le diluant contenant de la gélatine en boîtes réfrigérantes spéciales, puis on a effectué de nouveaux essais.

### Résultats, comparaison :

Les résultats des analyses microscopiques ont démontré dans presque tous les cas l'avantage du diluant de sperme contenant de la gélatine, par rapport aux autres diluants. Avec ce type de diluant, il y avait davantage de spermatozoïdes survivants, présentant une mobilité correcte.

Les résultats des inséminations sont résumés dans le tableau ci-après.

### Résultats comparés d'inséminations effectuées avec du sperme frais de lapin et avec du sperme de lapin dilué dans un diluant contenant de la gélatine après une durée de conservation de deux à trois jours.

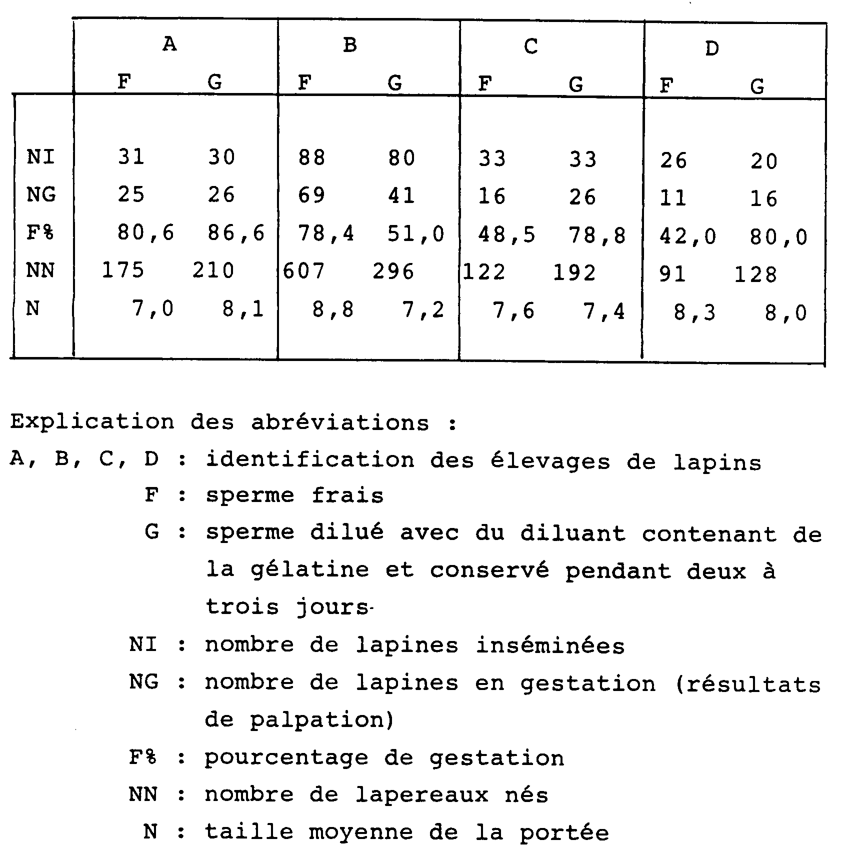

Il ressort du tableau ci-dessus que le sperme dilué avec un diluant contenant de la gélatine a conservé son pouvoir fécondant après deux à trois jours de conservation, et que son utilisation a apporté des résultats identiques à ceux obtenus avec du sperme fraîchement dilué.

Il est bien entendu que la présente invention n'a été décrite qu'à titre explicatif mais nullement limitatif et qu'on pourra y apporter toute modification utile, notamment dans le domaine des équivalences techniques, sans sortir de son cadre.

## Revendications

1. Procédé de dilution et de conservation du sperme de lapin, du type dans lequel on prépare un diluant liquide citraté contenant, pour 100 ml d'eau distillée stérile, environ 2,00 à 2,10 g de citrate de trisodium, 1,3 à 1,6 g de glucose D et 5 à 15 ml de jaune d'oeuf battu séparément, on ajoute au diluant liquide citraté environ 1 à 3 ml de gélatine ordinaire mise à l'état liquide par chauffage préalable jusqu'à environ 45 à 55°C, on laisse la température du mélange liquide s'abaisser jusqu'à environ 34 à 40°C et l'on ajoute alors le sperme de lapin fraîchement prélevé, caractérisé par le fait qu'on laisse alors la température dudit mélange liquide de sperme et de diluant s'abaisser jusqu'à environ 28 à 37°C, puis on fait barboter pendant quelques secondes du dioxyde de carbone dans le mélange liquide, à raison d'environ 5 à 15 cm³ de dioxyde de carbone pour 100 ml de sperme dilué puis, après barbotage, on ferme immédiatement le récipient contenant le sperme de lapin dilué et on laisse enfin le mélange se refroidir jusqu'à atteindre une température de stockage ou de transport d'environ 0 à 15°C, pour laquelle la gélatine ajoutée s'est entièrement solidifiée en gelée, fixant ainsi les spermatozoïdes mobiles, de sorte qu'ils ne perdent pas d'énergie et conservent leur pouvoir fécondant, tandis que le dioxyde de carbone élimine l'oxygène contenu dans le mélange à l'état liquide et favorise ainsi la conservation du sperme en condition d'anaérobie pendant 72 heures, la diminution de l'oxygène disponible entraînant un ralentissement de l'activité métabolique des spermatozoïdes tout en assurant la stabilité chimique du milieu à une valeur proche du pH normal du sperme de lapin.

2. Procédé suivant la revendication 1, caractérisé par le fait qu'on prépare un diluant liquide citraté contenant de préférence environ 2,03 g de citrate de trisodium, 1,5 g de glucose D et 10 ml de jaune d'oeuf pour 100 ml d'eau distillée stérile.

3. Procédé suivant la revendication 1, caractérisé par le fait qu'on ajoute au diluant liquide citraté de préférence environ 2 ml de gélatine ordinaire liquéfiée de préférence à environ 50°C.

4. Procédé suivant la revendication 1, caractérisé par le fait qu'on laisse la température du mélange liquide s'abaisser de préférence jusqu'à environ 37°C, qui est la température moyenne normale du sperme de lapin fraîchement prélevé.

5. Procédé suivant la revendication 1, caractérisé par le fait qu'après lui avoir fait subir un barbotage avec du dioxyde de carbone, on stocke ou on transporte le mélange solidifié en gelée, de préférence à une température comprise entre 5 et 10°C.

6. Procédé suivant l'une quelconque des revendications 1 à 5, caractérisé par le fait qu'après avoir ajouté le sperme de lapin au mélange liquide dont la température est encore d'environ 34 à 40°C et de préférence d'environ 37°C, on laisse la température du mélange liquide s'abaisser jusqu'à une température qui est de préférence d'environ 30 à 35°C, température à laquelle on réalise le barbotage du dioxyde de carbone.

7. Procédé suivant la revendication 6, caractérisé par le fait qu'on fait barboter le dioxyde de carbone dans le mélange liquide, de préférence à raison d'environ 10 cm³ pour 100 ml de sperme dilué.

8. Procédé suivant la revendication 7, caractérisé par le fait qu'on fait barboter le dioxyde de carbone dans le mélange liquide au moyen d'une seringue.

## Claims

1. Method for diluting and preserving rabbit semen, of the type in which a citrated liquid diluent is prepared, containing, for 100 ml of sterile distilled water approximately 2.0 to 2.10 g of trisodium citrate, 1.3 to 1.6 g of glucose D and 5 to 15 ml of yolk of egg whisked separately, 1 to 3 ml of common gelatine brought to the liquid state by previous heating upto about 45 to 55° C is added to the diluent, the temperature of the liquid mixture is let to fall to about 34 to 40° C and the freshly extracted semen is then added, characterised by the fact that the temperature of the said liquid mixture of semen and diluent is let to fall to about 28 to 37° C, then carbon dioxyde is brought to bubble during a few seconds in the mixture, at a rate of approximately 5 to 15 cm³ of carbon dioxyde per 100 ml of diluted semen, then after bubbling, the vessel containing the diluted rabbit semen is immediately closed, and last the mixture is let for cooling to reach a storage or transportation temperature of about 0 to 15° C, for which the added gelatine has completely solidified into jelly, thus fixing the mobile spermatozoa, so that they do not lose energy and preserve their fecundation power, whereas the carbon dioxyde suppresses the oxygen contained in the mixture at the liquid state and thus sponsors the preserving of the semen in an anaerobic condition during 72 hours, the reduction of the oxygen available entailing a slowing down of the metabolic activity of the spermatozoa while assuring the chemical stability of the medium at a value close to the normal pH of the rabbit semen.

2. Method according to claim 1, characterised by the fact that a citrated liquid diluent containing preferably approximately 2.03 g of trisodium citrate, 1.5 g of glucose D and 10 ml of yolk of egg per 100 ml of sterile distilled water is prepared.

3. Method according to claim 1, characterised by the fact that preferably approximately 2 ml of common gelatine preferably liquified at about 50° C is added to the citrated liquid diluent.

4. Method according to claim 1, characterised by the fact that the temperature of the liquid mixture is let to fall preferably to about 37° C, which is the normal average temperature of the rabbit semen freshly extracted.

5. Method according to claim 1, characterised by the fact that having been submitted to a bubbling with carbon dioxyde, the mixture solidified into jelly is stored or transported preferably at a temperature ranging between 5 to 10° C.

6. Method according to anyone of the preceding claims 1 to 5, characterised by the fact that after rabbit semen has been added to the liquid mixture the temperature of which is still about 34 to 40° C and preferably 37° C, the temperature of the liquid mixture is let to fall to a temperature preferably of 30 to 35° C, at which temperature bubbling of the carbon dioxyde is carried out.

7. Method according to claim 6, characterised by the fact that carbon dioxyde bubbles in the liquid mixture, preferably at a rate of about 10 cm³ per 100 ml of diluted semen.

8. Method according to claim 7, characterised by the fact that carbon dioxyde is brought to bubble in the liquid mixture, by means of a syringe.

## Patentansprüche

1. Verfahren zur Verdünnung und Konservierung von Kaninchensperma, derart, daß ein flüssiges zitrathaltiges Verdünnungsmittel, welches 100 ml steriles destilliertes Wasser, ca. 2,00 bis 2,10 g Trinatriumzitrat, 1,3 bis 1,6 g D-Glukose und 5 bis 15 ml getrennt geschlagenes Eigelb enthält, zubereitet wird, diesem flüssigen zitrathaltigen Verdünnungsmittel ca. 1 bis 3 ml einer gewöhnlichen, durch vorheriges Erwärmen auf ca. 45 bis 55°C verflüssigten Gelatine zugesetzt werden, wonach man die Temperatur der flüssigen Mischung auf ca. 34 bis 40°C abkühlen läßt und das frisch entnommenen Kaninchensperma hinuzfügt, **dadurch gekennzeichnet**, daß man die Temperatur der flüssigen Sperma- und Verdünnungsmittelmischung dann auf ca. 28 bis 37°C abkühlen läßt und sodann während einiger Sekunden Kohlendioxid im Verhältnis von ca. 5 bis 15 cm³ Kohlendioxid für 100 ml verdünnten Spermas in die flüssige Mischung einbläst und, danach, den das verdünnte Kaninchensperma enthaltenden Behälter sofort abschließt und schließlich die Mischung bis auf Lager- bzw. Transporttemperatur von ca. 0 bis 15°C abkühlen läßt, eine Temperatur bei der die hinzugefügte Gelatine vollständig als Gel erstarrt ist und dadurch die beweglichen Spermien fixiert, so daß sie keine Energie verlieren und ihr Befruchtungsvermögen bewahren, wobei das Kohlendioxid den in der flüssigen Mischung enthaltenen Sauerstoff eliminiert und somit die Konservierung des Spermas in anaerober Umgebung während 72 Stunden fördert, da durch die Verminderung des verfügbaren Sauerstoffs eine Verlangsamung der metabolischen Aktivität der Spermien unter Wahrung der chemischen Stabilität des Mediums bei einem dein normalen Weit des Kaninchenspermas nahekommenden pH-Wert bewirkt wird.

2. Verfahren nach Anspruch 1, **dadurch gekennzeichnet**, daß man ein flüssiges Verdünnungsmittel zubereitet, welches vorzugsweise ca. 2,03 g Trinatriumzitrat, 1,5 g D-Glukose und 10 ml Eigelb für 100 ml steriles destilliertes Wasser enthält.

3. Verfahren nach Anspruch 1, **dadurch gekennzeichnet**, daß man dem flüssigen zitrathaltigen Verdünnungsmittel vorzugsweise ca. 2 ml einer gewöhnlichen, bei einer Temperatur von vorzugsweise ca. 50°C verflüssigten Gelatine hinzufügt.

4. Verfahren nach Anspruch 1, **dadurch gekennzeichnet**, daß man die Temperatur der flüssigen Mischung vorzugsweise auf ca. 37°C - die normale Durchschnittstemperatur des frisch entnommenen Kaninchenspermas - abkühlen läßt.

5. Verfahren nach Anspruch 1, **dadurch gekennzeichnet**, daß man nach dem Einblasen von Kohlendioxid die als Gel erstarrte Mischung vorzugsweise bei einer Temperatur zwischen 5 und 10°C lagert bzw. transportiert.

6. Verfahren nach einem beliebigen Anspruch 1 bis 5, **dadurch gekennzeichnet**, daß nachdem der flüssigen Mischung, deren Temperatur noch ca. 34 bis 40°C und vorzugsweise ca. 37°C beträgt, das Kaninchensperma hinzugefügt wurde, man die Temperatur der flüssigen Mischung vorzugsweise auf ca. 30 bis 35°C abkühlen läßt und das Kohlendioxyd dann bei dieser Temperatur einbläst.

7. Verfahren nach Anspruch 6, **dadurch gekennzeichnet**, daß man das Kohlendioxyd in die flüssige Mischung vorzugsweise in einem Verhältnis von ca. 10 cm³ für 100 ml verdünnten Spermas einbläst.

8. Verfahren nach Anspruch 7, **dadurch gekennzeichnet**, daß man das Kohlendioxyd mittels einer Spritze in die flüssige Mischung einbläst.
